# EUROPEAN PATENT SPECIFICATION

(11) **EP 4 452 224 B1**
(45) Date of publication and mention of the grant of the patent: **07.10.2026**
(21) Application number: 22843294.4
(22) Date of filing: 21.12.2022
(51) Int. Cl.: A61K 9/00, A61K 9/08, A61K 47/18, A61K 47/40, A61K 31/517, A61P 31/12, A61P 31/22

(54) **PHARMACEUTICAL COMPOSITIONS COMPRISING 2-[(4S)-8-FLUORO-2-[4-(3-METHOXYPHENYL)PIPERAZIN-1-YL]-3-[2-METHOXY-5-(TRIFLUOROMETHYL)PHENYL]-4H-QUINAZOLIN-4-YL]ACETATE AND POTASSIUM IONS**
PHARMAZEUTISCHE ZUSAMMENSETZUNGEN MIT 2-[(4S)-8-FLUOR-2-[4-(3-METHOXYPHENYL)PIPERAZIN-1-YL]-3-[2-METHOXY-5-(TRIFLUORMETHYL)PHENYL]-4H-CHINAZOLIN-4-YL]ACETAT UND KALIUMIONEN
COMPOSITIONS PHARMACEUTIQUES COMPRENANT DE L'ACÉTATE DE 2-[(4S)-8-FLUORO-2-[4-(3-MÉTHOXYPHÉNYL)PIPERAZIN-1-YL]-3-[2-MÉTHOXY-5-(TRIFLUOROMÉTHYL)PHÉNYL]-4H-QUINAZOLIN-4-YL] ET DES IONS DE POTASSIUM

(30) Priority: 21.12.2021 EP 21216336
(43) Date of publication of application: 30.10.2024
(73) Proprietor: AIC246 AG & Co. KG, 42117 Wuppertal (DE)
(72) Inventor: BUSCHMANN, Helmut, 52076 Aachen (DE); GOLDNER, Thomas, 42553 Velbert (DE); CERÓN BERTRAN, Jordi Carles, 43761 La Pobla de Montornes (ES)
(74) Representative: Kilger, Ute
(86) International application number: PCT/EP2022/087245
(87) International publication number: WO 2023/118300

(56) References cited:
- WO-A1-2013/127971
- WO-A1-2021/170875
- WO-A1-2021/258206
- US-A1- 2021 369 619
- ANONYMOUS: "Letermovir", MONOGRAPH FOR PROFESSIONALS, 18 April 2021 (2021-04-18), pages 1 - 17, XP055909106, Retrieved from the Internet <URL:https://web.archive.org/web/20210418202741/https://www.drugs.com/monograph/letermovir.html> [retrieved on 20220405]

## Description

The present invention relates to new stable pharmaceutical compositions comprising 2-[(4S)-8-fluoro-2-[4-(3-methoxyphenyl)piperazin-1-yl]-3-[2-methoxy-5-(trifluoromethyl)phenyl]-4H-quinazolin-4-yl]acetatic acid, also known as letermovir, and potassium ions that are suitable for oral and intravenous application and for injection, wherein the potassium ions are contained in the form of a solution of potassium hydroxide (KOH).

Said pharmaceutical compositions are essentially free from particular complexing solubilizing agents, such as PEG, cyclodextrin, lysine, arginine, in particular HPBCD. Said formulations are suitable for use in methods of treatment of viral diseases, in particular human cytomegalovirus (hereinafter HCMV) infections. The invention also relates to methods of preparation of said pharmaceutical compositions.

### Background

Cytomegalovirus (CMV) is a common opportunistic infection that causes significant morbidity and preventable mortality after solid-organ and allogeneic hematopoietic stem cell transplantation.

HCMV is a species of virus that belongs to the viral family known as *Herpesviridae* or herpes viruses. It is typically abbreviated as HCMV and is alternatively known as human herpesvirus-5 (HHV-5). Within *Herpesviridae,* HCMV belongs to the *Betaherpesvirinae* subfamily, which also includes cytomegaloviruses from other mammals.

Letermovir is known as a highly active drug for addressing HCMV infection and extensively described in Lischka et al., In Vitro and In Vivo Activities of the Novel Anticytomegalovirus Compound Letermovir. Antimicrob. Agents Chemother. 2010, 54: p.1290-1297*,* and Kaul et al., First report of successful treatment of multidrug-resistant cytomegalovirus disease with the novel anti-CMV compound Letermovir. Am. J. Transplant. 2011, 11:1079-1084*;* as well as Marschall et al., In Vitro Evaluation of the Activities of the Novel Anticytomegalovirus Compound Letermovir against Herpesviruses and Other Human Pathogenic Viruses. Antimicrob. Agents Chemother. 2012, 56:1135-1137*.*

The precise chemical name of letermovir is 2-[(4S)-8-fluoro-2-[4-(3-methoxyphenyl)piperazin-1-yl]-3-[2-methoxy-5-(trifluoromethyl)phenyl]-4H-quinazolin-4-yl]acetic acid, and the chemical structure of letermovir is depicted below:

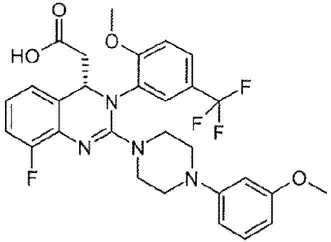

Letermovir was developed as an antiviral agent, in particular for the treatment, prevention, or prophylaxis of infections caused by the human cytomegalovirus (HCMV), and is disclosed in International Publication No. WO 2004/096778. In addition, salts of 2-[(4S)-8-fluoro-2-[4-(3-methoxyphenyl)piperazin-1-yl]-3-[2-methoxy-5-(trifluoromethyl)phenyl]-4H-quinazolin-4-yl]acetic acid were also prepared, as described in International Publication No. WO 2013/127971.

Liquid pharmaceutical formulations comprising amorphous letermovir are described in International Publication No. WO 2013/127970 which relates to a pharmaceutical composition that can be used in particular for intravenous administration that contains letermovir, that has long-term stability and can be stored, and that in addition has a substantially physiological pH. It has further been discovered that such compositions can be lyophilized in order to obtain a stable, solid pharmaceutical composition that can be reconstituted in a simple manner for injection purposes, e.g. by adding water, as a result of which, in turn, a stable pharmaceutical composition, e.g. for intravenous administration, can be obtained.

There remains a need, however, for pharmaceutical compositions comprising letermovir having long-term stability at substantially physiological pH, that are suitable for use in subjects of all ages in the need of solid-organ transplantationand allogenic hematopoietic stem cell transplantation.

### Description of the invention

In a first aspect, the present invention relates to a pharmaceutical composition comprising letermovir of formula (I), and potassium ions 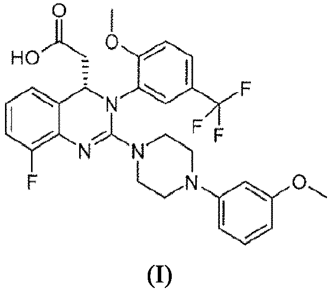 wherein the pharmaceutical composition
- comprises the potassium ions in a molar ratio to letermovir in the range of from 0.80 to < 1.00 : 1.00, preferably of from 0.88 to < 1.00 : 1.00, more preferably of from 0.90 to < 1.00 : 1.00; and
- is capable of exhibiting a pH in the range of from 7 to 8, when said pharmaceutical composition is dissolved in water in a concentration range of from 1 to 100 mg/mL with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH).

With the molar ratio of the potassium ions to letermovir in the range of from 0.80 to < 1.00 : 1.00, preferably of from 0.88 to < 1.00 : 1.00, more preferably of from 0.90 to < 1.00 : 1.00, letermovir exhibits an improved solubility and is present in a concentration sufficient to achieve the desired therapeutic effect without the need to use any further solubilizers, in particular complexing solubilizing agents such as cyclodextrins. In addition, the pharmaceutical composition which comprises the potassium ions in said ratio, has a substantially physiological pH and exhibits long-term stability.

It has been further discovered that said pharmaceutical composition can be obtained in a form of a lyophilizate that can be fully reconstituted in a parenterally acceptable diluent, such as water, glucose aqueous solution or Ringer's lactate solution. When reconstituted, said lyophilizate exhibits a pH in the range of from 7 to 8, preferably from 7.4 to 7.8, if Letermovir is present in a concentration range of from 1 to 100 mg/mL, preferably of from 20 to 100 mg/mL in said reconstituted solution. The pH of said reconstituted solution remains stable with molar ratio of the potassium ions to letermovir in the range of from 0.80 to < 1.00 : 1.00, preferably of from 0.88 to < 1.00 : 1.00, more preferably of from 0.90 to < 1.00 : 1.00, and is in the physiological range of from 7 to 8, preferably of from 7.4 to 7.8, what is a clear evidence of a surprising self-buffering effect of the potassium ions in the given ranges . The obtained reconstituted solutions exhibit a long-term stability.

In another aspect, the present invention relates to a method of producing of said pharmaceutical compositions, comprising the following steps:
i) providing a solution of letermovir and potassium ions, wherein the molar ratio of potassium ions to letermovir is in the range of from 0.80 to < 1.00 : 1.00, preferably of from 0.88 to < 1.00 : 1.00, more preferably of from 0.90 to < 1.00 : 1.00; and optionally at least one excipient selected from the group consisting of a carbohydrate, in particular sucrose or mannitol, an amino acid, in particular phenylalanine, a polyalkoxy compound, in particular a poloxamer, more particular poloxamer 188, and a polyvinylpyrrolidone (PVP), in particular PVP PF12, wherein the potassium ions are provided in the form of a KOH solution, preferably an aqueous KOH solution;
ii) if needed adjusting the pH of the solution obtained in step i) to a range of from 7 to 8 preferably with HCl;
iii) optionally filtering said solution.

In particular, the method according to the invention may further comprise the subsequent steps of freeze-drying the solution obtained in step iii above, to provide a lyophilizate and optionally reconstituting the lyophilizate in a first parenterally acceptable diluent to provide a reconstituted solution in a concentration range of from 1 to 100 mg/mL, preferably 20 to 100 mg/mL, with respect to letermovir and optionally further diluting said reconstituted solution with a second parenterally acceptable diluent to a final concentration which is acceptable for injection or infusion, and wherein said first and said second parenterally acceptable diluents can be the same or different.

Another aspect of the present invention relates to the pharmaceutical compositions described herein for use in a method of treatment and/or prevention of diseases, in particular of viral infections, preferably human cytomegalovirus (HCMV) infections or infections with another member of the herpes viridae group.

In particular, the pharmaceutical compositions according to the present invention are suitable for treatment of neonates, subjects in the need of particular solid-organ transplantation, e.g. subjects with kidney damages and subjects in need of allogenic hematopoietic stem cell transplantation.

### Detailed description

It is noted that the term "comprising" also encompasses the meaning "consisting of", e.g., a group of members comprising said members also encompasses a group of members consisting only of these members.

The term "room temperature" as used herein, is synonymous to the term "standard room temperature" and refers to a temperature in the range of from 19 °C to 26 °C. For example, "stirring at room temperature" means "stirring at a temperature in the range of from 19 °C to 26 °C".

Within the scope of the invention the term "stability" is understood to mean not only the chemical stability of the constituents of the pharmaceutical composition, in particular, the active substance, but also the physicochemical stability of the composition itself. In particular, the composition according to the invention must be stable against precipitation of the constituents.

In this context, the term "stability" means that at 2 °C to 8 °C, or at 25 °C or at 40 °C the pharmaceutical compositions according to the invention contain a minimum proportion of >90%, preferably >95%, and more preferably >98% of the active substance for a storage period of at least one month, preferably at least three months, even more preferably at least 6 months, even more preferably 12 months, even more preferably 18 months, and most preferred at least 36 months, when said liquid pharmaceutical compositions are measured according to the HPLC method of the present invention.

A cyclodextrin according to the invention is understood to be any modified or non-modified cyclodextrin, in particular selected from α-cyclodextrins, β-cyclodextrins or γ-cyclodextrins. The examples of modified β-cyclodextrins include, in particular, hydroxyalkyl-β-cyclodextrins, e.g. hydroxymethyl-β-cyclodextrins, hydroxyethyl-β-cyclodextrins or hydroxypropyl-β-cyclodextrins, alkyl-hydroxyalkyl-β-cyclodextrins, e.g. methyl-hydroxypropyl-β-cyclodextrins or ethyl-hydroxypropyl-cyclodextrins or sulfoalkyl-cyclodextrins. Hydroxypropyl-β-cyclodextrins are available in various degrees of substitution, in particular 2-hydroxypropyl-β-cyclodextrin is available as Cavasol ^{®} W7 HP, Cavitron ^{®} W7 HP5 and Cavitron ^{®} W7 HP7.

As used herein the term "complexing solubilizing agents" refers to the compounds which enhance solubility of the active ingredient of the pharmaceutical composition of the invention by forming coordination bonds between said compound and the molecule of the active ingredient, in particular in an aqueous solution, i.e. by actually and detectably forming a complex with the active ingredient of the pharmaceutical composition of the invention. The non-limiting examples of complexing solubilizing agents include non-polymeric solubilizers, such as lysine or arginine, and polymeric solubilizers, such as PEG or cyclodextrins.

As used herein the term "parenterally acceptable diluents", "parenteral admixture diluents" and "commercial diluents" refer to any liquid material which is used to dilute an active ingredient, which is suitable for administration to a subject by a route other than topical or oral. Examples of parenteral routes include intramuscular, intravascular (including intraarterial or intravenous), intraorbital, retrobulbar, intranasal, intrathecal, intraventricular, intraspinal, intraperitoneal, intrapulmonary, intracisternal, intracapsular, intrasternal, peribulbar, or intralesional administration. Examples of parenterally acceptable diluents include water, glucose aqueous solution or Ringer's lactate solution. Within the application the terms "commercial diluents", "parenteral admixture diluents" and "parenterally acceptable diluents" have the same meaning and are used interchangebly.

As used herein, the term "carbohydrate" refers to compounds that are polyhydroxy aldehydes or ketones, or substances that yield such compounds on hydrolysis. Some carbohydrates may further contain nitrogen, phosphorous, or sulfur. Examples of carbohydrates include monosaccharides, disaccharides, oligosaccharides, and polysaccharides, in particular sucrose and mannitol.

As used herein, the term "amino acid" refers to any of the twenty naturally occurring amino acids or their synthetic analogs with unnatural side chains and including both D and L optical isomers. The examples of amino acids include, in particular, alanine and phenylalanine.

As used herein, the term "polyalkoxy compounds" refers to the polymeric compounds in which the repeating units represent alkyl groups having straight or brached chain linked to an oxygen atom. The examples of polyalkoxy compounds include poloxamers, in particular, poloxamer 188.

Within the scope of the present invention the terms "obtained by" and "obtainable by" have the same meaning and are used interchangeably.

Within the scope of the present invention the term "equivalents" is understood to mean "molar equivalents".

As used herein the term "aqueous solution" refers to liquid homogeneous mixtures comprising water.

As used herein, the terms "lyophilization" and "freeze-drying" are used interchangeably and mean a process by which a desired product containing a solvent, in particular water, is cooled to a sufficient temperature, in particular by using liquid nitrogen or cooled shelves, at which a portion or all of the solvent is frozen and the frozen solvent is further removed by one or more drying steps, in particular by removal of unbound solvent by sublimation and desorption. The terms "lyophilizate" and "freeze-dried product" refer to the product obtained by freeze-drying and are used interchangeably throughout the application.

As used herein, the term "reconstitution" or "reconstituting" refers to a process of dissolving a lyophilizate in a diluent, preferably in a parenterally acceptable diluent, in particular water. The term "reconstituted solution" refers to the product obtained by reconstitution.

As used herein the term "treatment" or "treating" is defined as the application or administration of a therapeutic agent i.e., letermovir (alone or in combination with another pharmaceutical agent) to a subject, or application or administration of a therapeutic agent to an isolated tissue or cell line from a subject who has an HCMV infection, a symptom of HCMV infection, or the potential to develop an HCMV infection with the purpose to cure, heal, alleviate, relieve, alter, remedy, ameliorate, improve or affect the HCMV infection, the symptoms of HCMV infection or the potential to develop an HCMV infection. Such treatments may be specifically tailored or modified based on knowledge obtained from the field of pharmacogenomics.

As used herein the term "prevent", "preventing" or "prevention" means no disorder or disease development if none had occurred, or no further disorder or disease development if there had already been development of the disorder or disease. Also considered is the ability of one to prevent some or all of the symptoms associated with the disorder or disease. Prevention of diseases encompasses prophylaxis of diseases.

As used herein the term "subject" refers to a human or a non-human mammal. Non-human mammals include for example livestock and pets such as ovine, bovine, porcine, feline, canines and murine mammals. Preferably the subject is human. In one embodiment, the subject is a human infant. In a preferred embodiment, the subject is a human neonate. In another preferred embodiment, the subject is a subject in the need of particular solid-organ transplantation, e.g., a subject with kidney damages and a subject in need of allogenic hematopoietic stem cell transplantation.

As used herein the term "pharmaceutically acceptable" refers to a material such as a carrier or diluent which does not abrogate the biological activity or properties of the compound and is relatively non-toxic, i.e., the material may be administered to a subject without causing undesirable biological effects or interacting in a deleterious manner with any of the components of the composition in which it is contained.

As used herein the term "essentially free" refers to a content of less than 5 mole %.

The subject-matter of the present invention relates to a pharmaceutical composition comprising letermovir of formula (I), and potassium ions 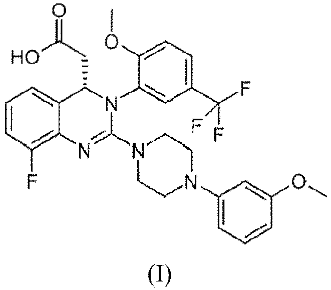 wherein the pharmaceutical composition
- comprises the potassium ions in a molar ratio to letermovir in the range of from 0.80 to < 1.00 : 1.00, preferably of from 0.88 to < 1.00 : 1.00, more preferably of from 0.90 to < 1.00 : 1.00; and
- is capable of exhibiting a pH in the range of from 7 to 8, preferably from 7.4 to 7.8, when said pharmaceutical composition is dissolved in water in a concentration range of from 1 to 100 mg/mL, preferably 20 to 100 mg/mL, with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH), preferably an aqueous solution of KOH.

The subject-matter of the present invention further relates to a pharmaceutical composition comprising letermovir of formula (I), and potassium ions 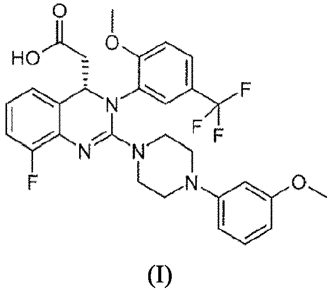 wherein the pharmaceutical composition
- comprises the potassium ions in a molar ratio to letermovir in the range of from 0.80 to < 1.00 : 1.00, preferably of from 0.88 to < 1.00 : 1.00, more preferably of from 0.90 to < 1.00 : 1.00; and
- is capable of exhibiting a pH in the range of from 7 to 8, preferably from 7.4 to 7.8, when said pharmaceutical composition is dissolved in a glucose aqueous solution, preferably 5% w/v glucose solution in water, in a concentration range of from 1 to 100 mg/mL, preferably 20 to 100 mg/mL, with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH), preferably an aqueous solution of KOH.

The subject-matter of the present invention further relates to a pharmaceutical composition comprising letermovir of formula (I), and potassium ions 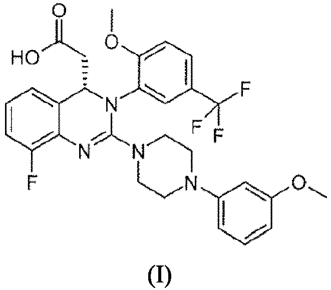 wherein the pharmaceutical composition
- comprises the potassium ions in a molar ratio to letermovir in the range of from 0.80 to < 1.00 : 1.00, preferably of from 0.88 to < 1.00 : 1.00, more preferably of from 0.90 to < 1.00 : 1.00; and
- is capable of exhibiting a pH in the range of from 7 to 8, preferably from 7.4 to 7.8, when said pharmaceutical composition is dissolved in Ringer's lactate solution in a concentration range of from 1 to 100 mg/mL, preferably 20 to 100 mg/mL, with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment, a pharmaceutical composition according to the invention is essentially free from a compound selected from the group consisting of PEG, lysine, arginine, and a cyclodextrin. In one embodiment a pharmaceutical composition according to the invention is essentially free from lysine. In another embodiment a pharmaceutical composition according to the invention is essentially free from arginine. In yet another embodiment a pharmaceutical composition according to the invention is essentially free from PEG. In yet another embodiment a pharmaceutical composition according to the invention is essentially free from a cyclodextrin. In a preferred embodiment a pharmaceutical composition according to the invention is essentially free from hydroxypropyl-beta-cyclodextrin. In another preferred embodiment a pharmaceutical composition according to the invention is essentially free from PEG, lysine, arginine and a cyclodextrin, in particular a hydroxypropyl-beta-cyclodextrin (HPBCD).

In one embodiment a pharmaceutical composition according to the invention is essentially free from complexing solubilizing agents, in particular essentially free from PEG, lysine, arginine, and a cyclodextrin, in particular a hydroxypropyl-beta-cyclodextrin (HPBCD).

In one embodiment, the content of complexing solubilizing agents in a pharmaceutical composition according to the invention is less than 5 mole %. In a preferred embodiment, the content of complexing solubilizing agents in a pharmaceutical composition according to the invention is less than 3 mole %. In a more embodiment, the content of complexing solubilizing agents in a pharmaceutical composition according to the invention is less than 1 mole %. In a more preferred embodiment, the content of complexing solubilizing agents in a pharmaceutical composition according to the invention is less than 0.5 mole %. Most preferred, the content of complexing solubilizing agents in a pharmaceutical composition according to the invention is less than 0.3 mole %.

In one embodiment the pharmaceutical composition according to the present invention comprises letermovir and potassium ions, wherein said pharmaceutical composition:
- comprises the potassium ions in a molar ratio to letermovir in the range of from 0.80 to < 1.00 : 1.00; and
- is capable of exhibiting a pH in the range of from 7 to 8, preferably from 7.4 to 7.8, when said pharmaceutical composition is dissolved in water in a concentration range of from 1 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.84 to < 1.00 : 1.00; and
- is capable of exhibiting a pH in the range of from 7 to 8, preferably from 7.4 to 7.8, when said pharmaceutical composition is dissolved in water in a concentration range of from 1 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In a preferred embodiment the pharmaceutical composition according to the present invention comprises letermovir and potassium ions, wherein said pharmaceutical composition:
- comprises the potassium ions in a molar ratio to letermovir in the range of from 0.88 to < 1.00 : 1.00; and
- is capable of exhibiting a pH in the range of from 7 to 8, preferably from 7.4 to 7.8, when said pharmaceutical composition is dissolved in water in a concentration range of from 1 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In a more preferred embodiment the pharmaceutical composition according to the present invention comprises letermovir and potassium ions, wherein said pharmaceutical composition:
- comprises the potassium ions in a molar ratio to letermovir in the range of from 0.90 to < 1.00 : 1.00; and
- is capable of exhibiting a pH in the range of from 7 to 8, preferably from 7.4 to 7.8, when said pharmaceutical composition is dissolved in water in a concentration range of from 1 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.80 to < 1.00 : 1.00; and
- is capable of exhibiting a pH in the range of from 7 to 8, preferably from 7.4 to 7.8, when said pharmaceutical composition is dissolved in water in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.84 to < 1.00 : 1.00; and
- is capable of exhibiting a pH in the range of from 7 to 8, preferably from 7.4 to 7.8, when said pharmaceutical composition is dissolved in water in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In a preferred embodiment the pharmaceutical composition according to the present invention comprises letermovir and potassium ions, wherein said pharmaceutical composition:
- comprises the potassium ions in a molar ratio to letermovir in the range of from 0.88 to < 1.00 : 1.00; and
- is capable of exhibiting a pH in the range of from 7 to 8, preferably from 7.4 to 7.8, when said pharmaceutical composition is dissolved in water in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In a more preferred embodiment the pharmaceutical composition according to the present invention comprises letermovir and potassium ions, wherein said pharmaceutical composition:
- comprises the potassium ions in a molar ratio to letermovir in the range of from 0.90 to < 1.00 : 1.00; and
- is capable of exhibiting a pH in the range of from 7 to 8, preferably from 7.4 to 7.8, when said pharmaceutical composition is dissolved in water in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.80 to < 1.00 : 1.00; and
- is capable of exhibiting a pH in the range of from 7 to 8, preferably from 7.4 to 7.8, when said pharmaceutical composition is dissolved in a glucose aqueous solution, preferably 5% w/v glucose solution in water, in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.84 to < 1.00 : 1.00; and
- is capable of exhibiting a pH in the range of from 7 to 8, preferably from 7.4 to 7.8, when said pharmaceutical composition is dissolved in a glucose aqueous solution, preferably 5% w/v glucose solution in water, in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.88 to < 1.00 : 1.00; and
- is capable of exhibiting a pH in the range of from 7 to 8, preferably from 7.4 to 7.8, when said pharmaceutical composition is dissolved in a glucose aqueous solution, preferably 5% w/v glucose solution in water, in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.90 to < 1.00 : 1.00; and
- is capable of exhibiting a pH in the range of from 7 to 8, preferably from 7.4 to 7.8, when said pharmaceutical composition is dissolved in a glucose aqueous solution, preferably 5% w/v glucose solution in water, in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.80 to < 1.00 : 1.00; and
- is capable of exhibiting a pH in the range of from 7 to 8, preferably from 7.4 to 7.8, when said pharmaceutical composition is dissolved in Ringer's lactate solution in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.84 to < 1.00 : 1.00; and
- is capable of exhibiting a pH in the range of from 7 to 8, preferably from 7.4 to 7.8, when said pharmaceutical composition is dissolved in Ringer's lactate solution in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.88 to < 1.00 : 1.00; and
- is capable of exhibiting a pH in the range of from 7 to 8, preferably from 7.4 to 7.8, when said pharmaceutical composition is dissolved in Ringer's lactate solution in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises letermovir and potassium ions, wherein said pharmaceutical composition:
- comprises the potassium ions in a molar ratio to letermovir in the range of from 0.90 to < 1.00 : 1.00; and
- is capable of exhibiting a pH in the range of from 7 to 8, preferably from 7.4 to 7.8, when said pharmaceutical composition is dissolved in Ringer's lactate solution in a concentration range of from 20 to 100 mg/mL with respect to letermovir; and
- is essentially free from complexing solubilizing agents selected from the group consisting of PEG, lysine, arginine, and a cyclodextrin, in particular a hydroxypropyl-beta-cyclodextrin (HPBCD), wherein the potassium ions are contained in the form of a solution of potassium hydroxide (KOH), preferably an aqueous solution of KOH.

In one embodiment the pharmaceutical composition according to the present invention comprises the potassium ions in the form of a solution of potassium hydroxide (KOH), preferably an aqueous solution of KOH, in a molar ratio to letermovir in the range of from 0.80 to < 1.00 : 1.00, preferably of from 0.81 to < 1.00 : 1.00, more preferably of from 0.82 to < 1.00 : 1.00, more preferably of from 0.83 to < 1.00 : 1.00, more preferably of from 0.84 to < 1.00 : 1.00, more preferably of from 0.85 to < 1.00 : 1.00, more preferably of from 0.86 to < 1.00 : 1.00, more preferably of from 0.87 to < 1.00 : 1.00, more preferably of from 0.88 to < 1.00 : 1.00, more preferably of from 0.89 to < 1.00 : 1.00, more preferably of from 0.90 to < 1.00 : 1.00.

In one embodiment the pharmaceutical composition according to the present invention is capable of exhibiting a pH in the range of from 7 to 8, when said pharmaceutical composition is dissolved in water in a concentration range of from 1 to 100 mg/mL with respect to letermovir. In a preferred embodiment the pharmaceutical composition according to the present invention is capable of exhibiting a pH in the range of from 7 to 8, when said pharmaceutical composition is dissolved in water in a concentration range of from 20 to 100 mg/mL with respect to letermovir.

In a preferred embodiment the pharmaceutical composition according to the present invention is capable of exhibiting a pH in the range of from 7.4 to 7.8, when said pharmaceutical composition is dissolved in water in a concentration range of from 1 to 100 mg/mL with respect to letermovir. In a more preferred embodiment the pharmaceutical composition according to the present invention is capable of exhibiting a pH in the range of from 7.4 to 7.8, when said pharmaceutical composition is dissolved in water in a concentration range of from 20 to 100 mg/mL with respect to letermovir.

In one embodiment the pharmaceutical composition according to the present invention is capable of exhibiting a pH in the range of from 7 to 8, when said pharmaceutical composition is dissolved in a glucose aqueous solution, preferably 5% w/v glucose solution in water, in a concentration range of from 20 to 100 mg/mL with respect to letermovir. In a preferred embodiment the pharmaceutical composition according to the present invention is capable of exhibiting a pH in the range of from 7.4 to 7.8, when said pharmaceutical composition is dissolved in a glucose aqueous solution, preferably 5% w/v glucose solution in water, in a concentration range of from 20 to 100 mg/mL with respect to letermovir.

In one embodiment the pharmaceutical composition according to the present invention is capable of exhibiting a pH in the range of from 7 to 8, when said pharmaceutical composition is dissolved in Ringer's lactate solution in a concentration range of from 20 to 100 mg/mL with respect to letermovir. In a preferred embodiment the pharmaceutical composition according to the present invention is capable of exhibiting a pH in the range of from 7.4 to 7.8, when said pharmaceutical composition is dissolved in Ringer's lactate solution in a concentration range of from 20 to 100 mg/mL with respect to letermovir.

In one embodiment a pharmaceutical composition comprising letermovir of formula (I), and potassium ions 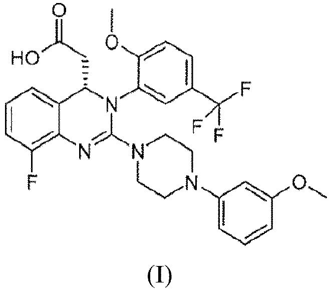 wherein the pharmaceutical composition
- comprises the potassium ions in a molar ratio to letermovir in the range of from 0.80 to < 1.00 : 1.00, preferably of from 0.88 to < 1.00 : 1.00, more preferably 0.90 to < 1.00 : 1.00; and
- is capable of exhibiting a pH in the range of from 7 to 8, preferably 7.4 to 7.8, when said pharmaceutical composition is dissolved in water in a concentration range of from 1 to 100 mg/mL with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH), preferably an aqueous solution of KOH, further comprises at least one pharmaceutical carrier or excipient.

In one embodiment a pharmaceutical composition comprising letermovir of formula (I), and potassium ions 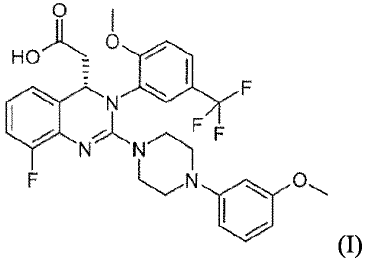 wherein the pharmaceutical composition
- comprises the potassium ions in a molar ratio to letermovir in the range of from 0.80 to < 1.00 : 1.00, preferably of from 0.88 to < 1.00 : 1.00, more preferably 0.90 to < 1.00 : 1.00; and
- is capable of exhibiting a pH in the range of from 7 to 8, preferably from 7.4 to 7.8, when said pharmaceutical composition is dissolved in a glucose aqueous solution, preferably 5% w/v glucose solution in water, in a concentration range of from 1 to 100 mg/mL, preferably 20 to 100 mg/mL, with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH), preferably an aqueous solution of KOH,
further comprises at least one pharmaceutical carrier or excipient.

In one embodiment a pharmaceutical composition comprising letermovir of formula (I), and potassium ions 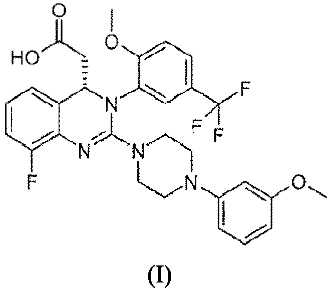 wherein the pharmaceutical composition
- comprises the potassium ions in a molar ratio to letermovir in the range of from 0.80 to < 1.00 : 1.00, more preferably of from 0.88 to < 1.00 : 1.00, more preferably of from 0.90 to < 1.00 : 1.00; and
- is capable of exhibiting a pH in the range of from 7 to 8, preferably from 7.4 to 7.8, when said pharmaceutical composition is dissolved in Ringer's lactate solution in a concentration range of from 1 to 100 mg/mL, preferably 20 to 100 mg/mL, with respect to letermovir; and
- is essentially free from complexing solubilizing agents selected from the group consisting of PEG, lysine, arginine, a cyclodextrin, in particular a hydroxypropyl-beta-cyclodextrin (HPBCD), wherein the potassium ions are contained in the form of a solution of potassium hydroxide (KOH), preferably an aqueous solution of KOH,
further comprises at least one pharmaceutical carrier or excipient.

In one embodiment a pharmaceutical composition according to the invention comprises at least one excipient selected from the group consisting of a carbohydrate, such as sucrose or mannitol; an amino acid, such as phenylalanine; a polyalkoxy compound, such as a poloxamer, more particular poloxamer 188; and a polyvinylpyrrolidone (PVP), such as PVP PF12. In a preferred embodiment said excipient is mannitol or sucrose or a combination thereof.

In one embodiment a pharmaceutical composition according to the invention is essentially free from complexing solubilizing agents.

In one embodiment a pharmaceutical composition according to the invention may contain an excipient which exhibits complexing solubilizing properties. In one embodiment such an excipient is a polyalkoxy compound, such as a poloxamer. In one embodiment, the poloxamer is poloxamer 188.

In one embodiment, the pharmaceutical composition according to the invention comprises a polyalkoxy compound, such as a poloxamer, such as poloxamer 188, and is essentially free from other complexing solubilizing agents.

In one embodiment the used excipients are suitable for administration to subjects in the need of particular solid-organ transplantation, e.g., subjects with kidney damages and subjects in need of allogenic hematopoietic stem cell transplantation. Non-limiting examples of such excipients include sucrose, mannitol, phenylalanine, and a poloxamer, such as poloxamer 188, and a polyvinylpyrrolidone (PVP), such as PVP PF12.

In one embodiment a pharmaceutical composition according to the invention further comprises a buffer, preferably Tris hydroxy aminomethane (Tris).

In one embodiment a pharmaceutical composition according to the invention further comprises HCl.

In one embodiment a pharmaceutical composition according to the invention represents a stability in accordance with ICH Q1A (R2) (Stability testing of new drug substances and drug products) covering the climate zones I to IV. In a preferred embodiment a pharmaceutical composition according to the invention is stable for at least one month. In a more preferred embodiment a pharmaceutical composition according to the invention is stable for at least three months. In a more preferred embodiment a pharmaceutical composition according to the invention is stable for at least 6 months. In a more preferred embodiment a pharmaceutical composition according to the invention is stable for at least 12 months. In a more preferred embodiment a pharmaceutical composition according to the invention is stable for at least 18 months. In a more preferred embodiment a pharmaceutical composition according to the invention is stable for at least 36 months.

In one embodiment a pharmaceutical composition according to the invention is in a solid form.

In a preferred embodiment said solid form of said pharmaceutical composition is a lyophilizate.

In one embodiment a pharmaceutical composition according to the invention is in a liquid form.

In a preferred embodiment said liquid form of a pharmaceutical composition according to the invention is an aqueous solution. In another preferred embodiment said liquid form of a pharmaceutical composition according to the invention is a solution in at least one parenterally acceptable diluent. Non-limiting examples of parenterally acceptable diluents include water, glucose aqueous solution and Ringer's lactate solution.

In one embodiment a pharmaceutical composition according to the invention is suitable for intravenous (IV) application or for injection.

The subject-matter of the present invention further relates to a method of producing the pharmaceutical composition according to the invention, comprising the following step:
i) providing a solution of letermovir and potassium ions, wherein the molar ratio of potassium ions to letermovir is in the range of from 0.80 to < 1.00 : 1.00, preferably 0.84 to < 1.00 : 1.00, more preferably of from 0.88 to < 1.00 : 1.00, more preferably of from 0.90 to < 1.00 : 1.00; and optionally at least one excipient selected from the group consisting of a carbohydrate, in particular sucrose or mannitol, an amino acid, in particular phenylalanine, a polyalkoxy compound, in particular a poloxamer, more particular poloxamer 188, and a polyvinylpyrrolidone (PVP), in particular PVP PF12, wherein the potassium ions are contained in the form of a solution of potassium hydroxide (KOH), preferably an aqueous solution of KOH.

In one embodiment the solution provided in step i above is a solution in a parenterally acceptable diluent, such as water.

In one embodiment providing the solution according to step i above comprises the following steps:
a-1) providing a suspension of letermovir in a parenterally acceptable diluent, in particular water;
b-1) adding KOH to the suspension obtained in step a-1 to provide a mixture;
c-1) optionally stirring the mixture obtained in step b-1 for at least 30 min.
d-1) optionally adding at least one excipient selected from the group consisting of a carbohydrate, in particular sucrose and mannitol, an amino acid, in particular phenylalanine, a polyalkoxy compound, in particular a poloxamer, more particular poloxamer 188, and a polyvinylpyrrolidone (PVP), in particular PVP PF12 to said mixture;
e-1) optionally stirring said mixture for at least 30 min.

In a preferred embodiment, an aqueous solution of KOH is added in step b-1.

In a preferred embodiment, the solution in step c-1 is stirred for at least 2 hours.

In a preferred embodiment, the solution in step e-1 is stirred for at least 2 hours.

In a preferred embodiment 0.80 to <1.00 equivalents of KOH with respect to letermovir are added in step b-1. In a more preferred embodiment 0.84 to <1.00 equivalents of KOH with respect to letermovir are added in step b-1. In a more preferred embodiment 0.88 to <1.00 equivalents of KOH with respect to letermovir are added in step b-1. In a more preferred embodiment 0.90 to <1.00 equivalents of KOH with respect to letermovir are added in step b-1.

In one embodiment 0.80 equivalents of KOH with respect to letermovir are added in step b-1.

In one embodiment 0.82 equivalents of KOH with respect to letermovir are added in step b-1.

In one embodiment 0.84 equivalents of KOH with respect to letermovir are added in step b-1.

In one embodiment 0.86 equivalents of KOH with respect to letermovir are added in step b-1.

In one embodiment 0.88 equivalents of KOH with respect to letermovir are added in step b-1.

In one embodiment 0.90 equivalents of KOH with respect to letermovir are added in step b-1.

In another embodiment the method for providing a solution according to step i comprises utilizing the following steps a-2 to e-2 in place of steps a-1 to e-1:
a-2) providing a solution of KOH in a parenterally acceptable diluent, in particular water;
b-2) adding letermovir to the solution obtained in step a-2 to provide a mixture;
c-2) optionally stirring the mixture obtained in step b-2 for at least 30 minl;
d-2) optionally adding at least one excipient selected from the group consisting of a carbohydrate, in particular sucrose and mannitol, an amino acid, in particular phenylalanine, a polyalkoxy compound, in particular a poloxamer, more particular poloxamer 188, and a polyvinylpyrrolidone (PVP), in particular PVP PF12 to said mixture;
e-2) optionally stirring said mixture for at least 30 minl.

In a preferred embodiment, the solution in step c-2 is stirred for at least 2 hours.

In a preferred embodiment, the solution in step e-2 is stirred for at least 2 hours.

In a preferred embodiment 1.25 to >1.00 equivalents of letermovir with respect to KOH are added in step b-2. In a more preferred embodiment 1.19 to >1.00 equivalents of letermovir with respect to KOH are added in step b-2. In a more preferred embodiment 1.14 to >1.00 equivalents of letermovir with respect to KOH are added in step b-2. In a more preferred embodiment 1.11 to >1.00 equivalents of letermovir with respect to KOH are added in step b-2.

In one embodiment 1.25 equivalents of letermovir with respect to KOH are added in step b-2.

In one embodiment 1.22 equivalents of letermovir with respect to KOH are added in step b-2.

In one embodiment 1.19 equivalents of letermovir with respect to KOH are added in step b-2.

In one embodiment 1.16 equivalents of letermovir with respect to KOH are added in step b-2.

In one embodiment 1.14 equivalents of letermovir with respect to KOH are added in step b-2.

In one embodiment 1.11 equivalents of letermovir with respect to KOH are added in step b-2.

In one embodiment the method of producing the pharmaceutical composition according to the present invention further comprises adjusting the pH of the solution obtained in step i to a range of from 7 to 8, preferably from 7.4 to 7.8. In one preferred embodiment said adjustment is performed by adding HCl. In a more preferred embodiment the pH of the solution obtained in step i is in the range of from 7 to 8, preferably from 7.4 to 7.8 and the pH adjustment is not necessary.

In one embodiment the solution obtained after the pH adjustment is optionally stirred for at least 10 min, preferably at least 30 min.

In one embodiment the method of producing the pharmaceutical composition according to the present invention optionally comprises filtering the solution obtained in step i. In one embodiment the method of producing the pharmaceutical composition according to the present invention optionally comprises filtering the solution obtained after adjustment of the pH of the solution obtained in step i above.

In one embodiment the method of producing the pharmaceutical composition according to the present invention further comprises freeze-drying the obtained solution to provide a lyophilizate.

In one embodiment the method of producing the pharmaceutical composition according to the present invention further comprises reconstituting the lyophilizate in a first parenterally acceptable diluent to provide a reconstituted solution in a concentration range of from 0.1 to 100 mg/mL with respect to letermovir and optionally further diluting said reconstituted solution with a second parenterally acceptable diluent to a final concentration which is acceptable for injection or infusion. Said first and said second parenterally acceptable diluents can be the same or different. In one embodiment said reconstituted solution exhibits a pH a range of from 7 to 8, preferably from 7.4 to 7.8, when letermovir is present in a concentration range of from 0.1 to 100 mg/mL in said reconstituted solution. In a preferred embodiment said reconstituted solution exhibits a pH in a range of from 7 to 8, preferably from 7.4 to 7.8, when letermovir is present in a concentration range of from 20 to 100 mg/mL in said reconstituted solution.

In one embodiment the final concentration which is acceptable for injection or infusion is in a range from 0.1 to 100 mg/mL. In another embodiment the final concentration which is acceptable for injection or infusion is in a range from 0.8 to 100 mg/mL. In another embodiment the final concentration which is acceptable for injection or infusion is in a range from 20 to 100 mg/mL. In another embodiment the final concentration which is acceptable for injection or infusion is in a range from 50 to 100 mg/mL. In another embodiment the final concentration which is acceptable for injection or infusion is in a range from 20 to 50 mg/mL. In a preferred embodiment the final concentration which is acceptable for injection or infusion is 0.8 mg/mL. In a preferred embodiment the method of producing the pharmaceutical composition according to the present invention comprises the following steps:
i) providing a solution of letermovir and potassium ions, wherein the molar ratio of potassium ions to letermovir is in the range of from 0.80 to < 1.00 : 1.00, preferably 0.84 to < 1.00 : 1.00, more preferably of from 0.88 to < 1.00 : 1.00, more preferably of from 0.90 to < 1.00 : 1.00; and optionally at least one excipient selected from the group consisting of a carbohydrate such as sucrose or mannitol; an amino acid such as phenylalanine; a polyalkoxy compound such as a poloxamer, particularly poloxamer 188; and a polyvinylpyrrolidone (PVP such as PVP PF12), wherein the potassium ions are contained in the form of a solution of potassium hydroxide (KOH), preferably an aqueous solution of KOH;
ii) if needed adjusting the pH of the solution obtained in step i to a range of from 7 to 8, preferably from 7.4 to 7.8, with a suitable organic and inorganic acid;
iii) optionally filtering the obtained solution.

In one embodiment of step ii the organic or inorganic acid is HCl.

In another preferred embodiment the method of producing the pharmaceutical composition according to the present invention comprises the following steps:
i) providing a solution of letermovir and potassium ions, wherein the molar ratio of potassium ions to letermovir is in the range of from 0.80 to < 1.00 : 1.00, preferably 0.84 to < 1.00 : 1.00, more preferably of from 0.88 to < 1.00 : 1.00, more preferably of from 0.90 to < 1.00 : 1.00; and optionally at least one excipient selected from the group consisting of a carbohydrate such as sucrose or mannitol; an amino acid such as phenylalanine; a polyalkoxy compound such as a poloxamer, particularly poloxamer 188; and a polyvinylpyrrolidone (PVP such as PVP PF12), wherein the potassium ions are contained in the form of a solution of potassium hydroxide (KOH), preferably an aqueous solution of KOH;
ii) if needed adjusting the pH of the solution obtained in step i to a range of from 7 to 8, preferably from 7.4 to 7.8, with a suitable organic and inorganic acid;
iii) optionally filtering the obtained solution;
iv) freeze-drying the obtained solution to provide a lyophilizate.

In one embodiment of step ii the organic or inorganic acid is HCl.

In another preferred embodiment the method of producing the pharmaceutical composition according to the present invention comprises the following steps:
i) providing a solution of letermovir and potassium ions, wherein the molar ratio of potassium ions to letermovir is in the range of from 0.80 to < 1.00 : 1.00, preferably 0.84 to < 1.00 : 1.00, more preferably of from 0.88 to < 1.00 : 1.00, more preferably of from 0.90 to < 1.00 : 1.00; and optionally at least one excipient selected from the group consisting of a carbohydrate such as sucrose or mannitol; an amino acid such as phenylalanine; a polyalkoxy compound such as a poloxamer, particularly poloxamer 188; and a polyvinylpyrrolidone (PVP), such as PVP PVP PF12, wherein the potassium ions are contained in the form of a solution of potassium hydroxide (KOH), preferably an aqueous solution of KOH;
ii) if needed adjusting the pH of the solution obtained in step i to a range of from 7 to 8, preferably from 7.4 to 7.8, with a suitable organic and inorganic acid;
iii) optionally filtering the obtained solution;
iv) freeze-drying the obtained solution to provide a lyophilizate;
v) reconstituting the lyophilizate in a first parenterally acceptable diluent to provide a reconstituted solution in a concentration range of from 1 to 100 mg/mL, preferably of from 20 to 100 mg/mL with respect to letermovir and optionally further diluting said reconstituted solution with a second parenterally acceptable diluent to a final concentration which is acceptable for injection or infusion, wherein said first and said second parenterally acceptable diluents can be identical or different from each other.

In one embodiment of step ii the organic or inorganic acid is HCl.

The above steps i to v do not necessarily signify a specific sequence or number of steps. However, preferably the steps of the method are implemented in the order as shown above.

Some of said steps may be optional and in some embodiments optional steps are not implemented. For example in one embodiment step ii may directly be followed by step iv without implementation of step iii. Also the above shown steps do not exclude additional steps that are not explicitly mentioned. For example, the solution obtained in step i and/or ii may be optionally stirred.

The subject-matter of the present invention further relates to a pharmaceutical composition, which is obtainable by any method disclosed herein.

The pharmaceutical compositions according to the invention may be used to produce drugs which are suitable for use in methods of preventing and/or treating infections with a representative of the Herpes viridae group, in particular a cytomegalovirus, in particular the human cytomegalovirus.

Further subject matter of the present invention the pharmaceutical compositions according to the invention for use in the method of treating and/or preventing diseases, preferably viral infections, in particular infections with the human cytomegalovirus (HCMV) or another representative of the Herpes viridae group.
said subject is selected from the group consisting of neonates, subjects in the need of particular solid-organ transplantation, e.g., subjects with kidney damages and subjects in need of allogenic hematopoietic stem cell transplantation.

In general, it has proven to be advantageous to administer the pharmaceutical compositions in such a way that about 0.001 to 10 mg per kg, preferably 0.01 to 5 mg per kg body weight of 2-[(4S)-8-fluoro-2-[4-(3-methoxyphenyl)piperazin-1-yl]-3-[2-methoxy-5-(trifluoromethyl)phenyl]-4H-quinazolin-4-yl]acetatic acid (letermovir) is administered.

Nevertheless, it may be necessary to deviate from the stated amounts of letermovir, namely depending on body weight, individual response to the active substance and the time and interval at which it is administered. For example, in certain cases it may be sufficient to administer less than the aforementioned minimum amount of letermovir, while in other cases the stated upper limit may be exceeded. When administering large amounts it may be recommendable to distribute these in several individual doses over the course of a day.

The invention will now be described in detail on the basis of non-restrictive examples.

Unless otherwise stated, the percentages given in the following tests and examples are weight percentages, parts are weight proportions, solvent ratios, dilution ratios and concentrations of liquid solutions relate, in each case, to the volume.

### Abbreviations

- API: active pharmaceutical ingredient
- h: hour(s)
- HCl: hydrochloric acid
- HEPES: (4-(2-hydroxyethyl)-1-piperazineethanesulfonic acid)
- HPBCD: hydroxypropyl-beta-cyclodextrin
- HPLC: high pressure liquid chromatography
- conc.: concentrated
- min.: minutes
- LAF: laminar air flow
- PEG: polyethylene glycol
- PDE: permissible daily exposures
- R_{T}: retention time (in HPLC)
- RP-HPLC: reversed phase high pressure liquid chromatography
- rpm: revolutions per minute
- rt: room temperature

### Analytical methods

### Visual inspection

The samples were inspected for the presence or absence of visible particles under gentle, manual, radial agitation for 5 seconds in front of a white background.

### pH

The pH value of the samples was measured with a calibrated pH meter EUTEGH CAKTON PH/Ion 510 Serial n° 172361 with a Polilyte lab electrode. The sample is stirred and the electrode is introduced. A measurement is performed until the pH value is stable. Between measurements the electrode is thoroughly rinsed with water. The pH measurements were performed with an analysis volume of ~1-2 mL and a defined temperature of 22 °C ± 3 °C. A 3-point calibration of the pH meter was performed on a daily basis, by using buffers with pH 7.00, pH 4.01 and pH 10.01 (Hamilton Duracal buffer).

### Reversed-phase high performance liquid chromatography (RP-HPLC)

RP-HPLC was used to determine the concentration of letermovir free base and potential degradation products.

Table 1 gives an overview of the eluents that were used for RP-HPLC analysis.

**Table 1: Eluents to be used for RP-HPLC analysis.**

| **Eluents** | **Preparation** |
|---|---|
| Eluent A: (0.1% formic acid in water) | Add 500 µl formic acid to a volumetric flask and fill up to 500 ml with highly purified water |
| Eluent B: (0.1% formic acid in methanol) | Add 500 µl formic acid to a volumetric flask and fill up to 500 ml with methanol |

The following parameters were used for the RP-HPLC method:
- Instrument:: Agilent Technologies 1200 series with a VWD G131413 detector
- Column:: Agilent Zorbax Eclipse XDB C-18, 150 x 4.6 mm, 5 µm
- Flow rate:: 1.0 ml/min
- Solvent A:: 0.1% formic acid in water
- Solvent B:: 0.1% formic acid in 100% methanol
- Stop time:: 26 minutes
- Injection volume:: 10 µl
- Column temperature:: 35 °C
- Wavelength:: 260 nm

Table 2 shows the gradient that was used for the RP-HPLC method.

**Table 2: Gradient applied during RP-HPLC analysis.**

| **Time [min]** | **Eluent B [%]** |
|---|---|
| 0.00 | 5.0 |
| 1.00 | 5.0 |
| 20.00 | 95.0 |
| 23.00 | 95.0 |
| 23.10 | 5.0 |
| 26.00 | 5.0 |

A calibration curve of the reference standard was used for the quantification of letermovir free base in solution.

The samples were diluted to approximately 2 mg/mL in water (corrected for letermovir free base in solution) and analyzed with an injection volume of 10 µl. Prior to injection, the diluted samples were filtered through a syringe filter (nylon, 0.45 µm).

Peak integration was performed manually for all API-related peaks. Peaks that were also present in blank or formulation buffer injections were neglected.

### Powder X-ray diffraction (PXRD)

Equipment: Powder diffraction patterns were acquired on a Bruker D8 Advance Series 2Theta/Theta powder diffraction system using CuKα1-radiation in transmission geometry. The system is equipped with a VÅNTEC-1 single photon counting PSD, a Germanium monochromator, fixed divergence slits and a radial soller. Software used: Data collection with DIFFRAC plus XRD Commander V.2.5.1, and evaluation with EVA V.5.0.0.22 (Bruker-AXS 2010-2018).

Sample preparation: Approximately 15 mg of non-manipulated sample were prepared in standard sample holders using two foils of polyacetate.

Measurement conditions: The samples were measured at room temperature in a range from 4° to 40° in 2Θ in a 0.1 hours measurement using an angular step of 0.049° and a time per step of 2787 s.

### Examples

### Example 1. Monitoring of solutions of letermovir free base with different equivalents of potassium hydroxide at different temperatures

### a) Initial drying

26 samples of letermovir free base were prepared by weighing the substance and drying in a vacuum oven at 90 °C (ca. 5 mbar) overnight in order to remove residual water and to avoid weighing errors to calculate the equivalents of KOH (Table 3).

Samples of ca. 80 mg and 300 mg were weighed to be dissolved in 4 mL and 3 mL, respectively, to prepare 20 mg/mL and 100 mg/mL solutions.

**Table 3. Initial drying process of 26 samples of letermovir free base**

| **Planned Letermovir Free Base concentration [mg/ml]** | **Temperature During Dissolution** | **Planned KOH/Letermovir Molar Ratio** | **Starting material, g** | **Sample lost, g** | **Final weight, g** |
|---|---|---|---|---|---|
| 20 | rt | 0.80 / 1 | 0.0809 | 0.0050 | 0.0759 |
| 20 | rt | 0.82 / 1 | 0.0807 | 0.0047 | 0.0760 |
| 20 | rt | 0.84 / 1 | 0.0818 | 0.0043 | 0.0775 |
| 20 | rt | 0.86 / 1 | 0.0801 | 0.0044 | 0.0757 |
| 20 | rt | 0.88 / 1 | 0.0810 | 0.0038 | 0.0772 |
| 20 | rt | 0.90 / 1 | 0.0816 | 0.0034 | 0.0782 |
| 20 | rt | 1 / 1 | 0.0808 | 0.0043 | 0.0765 |
| 100 | rt | 0.80 / 1 | 0.3021 | 0.0088 | 0.2933 |
| 100 | rt | 0.82 / 1 | 0.3080 | 0.0085 | 0.2995 |
| 100 | rt | 0.84 / 1 | 0.3080 | 0.0084 | 0.2996 |
| 100 | rt | 0.86 / 1 | 0.3052 | 0.0079 | 0.2973 |
| 100 | rt | 0.88 / 1 | 0.3031 | 0.0077 | 0.2954 |
| 100 | rt | 0.90 / 1 | 0.3035 | 0.0077 | 0.2958 |
| 100 | rt | 1 / 1 | 0.3032 | 0.0076 | 0.2956 |
| 20 | 40°C | 0.84 / 1 | 0.0803 | 0.0041 | 0.0762 |
| 20 | 40°C | 0.86 / 1 | 0.0800 | 0.0047 | 0.0753 |
| 20 | 40°C | 0.88 / 1 | 0.0809 | 0.0040 | 0.0769 |
| 20 | 60°C | 0.84 / 1 | 0.0802 | 0.0029 | 0.0773 |
| 20 | 60°C | 0.86 / 1 | 0.0808 | 0.0031 | 0.0777 |
| 20 | 60°C | 0.88 / 1 | 0.0808 | 0.0030 | 0.0778 |
| 100 | 40°C | 0.84 / 1 | 0.3064 | 0.0070 | 0.2994 |
| 100 | 40°C | 0.86 / 1 | 0.3070 | 0.0077 | 0.2993 |
| 100 | 40°C | 0.88 / 1 | 0.3016 | 0.0075 | 0.2941 |
| 100 | 60°C | 0.84 / 1 | 0.3010 | 0.0063 | 0.2947 |
| 100 | 60°C | 0.86 / 1 | 0.3028 | 0.0057 | 0.2971 |
| 100 | 60°C | 0.88 / 1 | 0.3033 | 0.0060 | 0.2973 |

### b) Preparation of suspensions/solutions and analysis of pH and solubility

Procedure: The corresponding amount of water and the respective equivalents of 1 M aqueous KOH was added to each sample. The suspensions were stirred at room temperature, at 40 °C or at 60 °C respectively. The pH, solubility and precipitation were monitored at 12h, 24h, 48h and 7 days (Table 4, Table 5, Table 6 and Table 7)

**Table 4. Preparation of samples of letermovir free base of 20 mg/mL in water with different equivalents of KOH at room temperature.**

| **Letermovir Free Base concentration [mg/ml]** | **KOH/Letermovir Molar Ratio** | **µL of 1M KOH_{aq}** | **mL of H₂O** | **pH 12h** | **pH 24h** | **pH 48h** | **pH 1w** | **Solubility** |
|---|---|---|---|---|---|---|---|---|
| 20 | 0.80 / 1 | 107 | 3.69 | 7.8 | 7.7 | 7.8 | 7.6 | a |
| 20 | 0.82 / 1 | 109 | 3.69 | 7.8 | 7.8 | 7.8 | 7.6 | a |
| 20 | 0.84 / 1 | 114 | 3.76 | 7.8 | 7.8 | 7.8 | 7.6 | a |
| 20 | 0.86 / 1 | 114 | 3.67 | 7.8 | 7.7 | 7.7 | 7.5 | a |
| 20 | 0.88 / 1 | 119 | 3.74 | 7.8 | 7.7 | 7.8 | 7.5 | a |
| 20 | 0.90 / 1 | 123 | 3.79 | 7.8 | 7.7 | 7.8 | 7.5 | a |
| 20 | 1 / 1 | 134 | 3.69 | 7.9 | 7.8 | 7.8 | 7.7 | b |

| | | | | | | | | |
|---|---|---|---|---|---|---|---|---|
| **a:** particles in suspension; **b:** particles in suspension, but after 24 h only above the solution (interphase air-water). | | | | | | | | |

**Table 5. Preparation of samples of letermovir free base of 100 mg/mL in water with different equivalents of KOH at room temperature.**

| **Letermovir Free Base concentration [mg/ml]** | **KOH/Letermovir Molar Ratio** | **µL of 1M KOH_{aq}** | **mL of H₂O** | **pH 12h** | **pH 24h** | **pH 48h** | **pH 1w** | **Solubility** |
|---|---|---|---|---|---|---|---|---|
| 100 | 0.80 / 1 | 410 | 2.52 | 7.5 | 7.4 | 7.6 | 7.3 | c |
| 100 | 0.82 / 1 | 429 | 2.57 | 7.5 | 7.4 | 7.6 | 7.3 | c |
| 100 | 0.84 / 1 | 439 | 2.56 | 7.5 | 7.5 | 7.6 | 7.3 | c |
| 100 | 0.86 / 1 | 447 | 2.53 | 7.5 | 7.5 | 7.7 | 7.4 | c |
| 100 | 0.88 / 1 | 454 | 2.50 | 7.7 | 7.7 | 7.8 | 7.5 | c |
| 100 | 0.90 / 1 | 465 | 2.49 | 7.7 | 7.6 | 7.7 | 7.4 | c |
| 100 | 1 / 1 | 516 | 2.44 | 7.9 | 7.8 | 7.9 | 7.7 | c |

| | | | | | | | | |
|---|---|---|---|---|---|---|---|---|
| **c:** clear solution. | | | | | | | | |

**Table 6. Preparation of samples of letermovir free base of 20 mg/mL in water with different equivalents of KOH at 40 °C and 60 °C.**

| **Letermovir Free Base concentration [mg/ml]** | **Temperature** | **KOH/ Letermovir Molar Ratio** | **µL of 1M KOH_{aq}** | **mL of H₂O** | **pH 12h** | **pH 24h** | **pH 48h** | **pH 1w** | **Solubility** |
|---|---|---|---|---|---|---|---|---|---|
| 20 | 40 °C | 0.84 / 1 | 112 | 3.70 | 7.8 | 7.7 | 7.8 | 7.8 | d |
| 20 | 40 °C | 0.86 / 1 | 113 | 3.65 | 7.7 | 7.7 | 7.8 | 7.7 | d |
| 20 | 40 °C | 0.88 / 1 | 118 | 3.73 | 7.7 | 7.6 | 7.8 | 7.7 | d |
| 20 | 60 °C | 0.84 / 1 | 113 | 3.75 | 7.6 | 7.6 | 7.7 | 7.6 | a |
| 20 | 60 °C | 0.86 / 1 | 117 | 3.77 | 7.6 | 7.6 | 7.8 | 7.6 | a |
| 20 | 60 °C | 0.88 / 1 | 120 | 3.77 | 7.6 | 7.5 | 7.8 | 7.6 | a |

| | | | | | | | | | |
|---|---|---|---|---|---|---|---|---|---|
| **d:** cloudy; **a:** particles in suspension, after 24 h few particles, after 48 h only above the solution and after 1 week, clear solution. suspension, after 24 h only above the solution and after 1 week, clear solution; **g:** cloudy; **h:** few particles in suspension, even after 1 week; **i:** clear solution. | | | | | | | | | |

**Table 7. Preparation of samples of letermovir free base of 100 mg/mL in water with different equivalents of KOH at 40 °C and 60 °C.**

| **Letermovir Free Base concentration [mg/ml]** | **Temperature** | **KOH/ Letermovir Molar Ratio** | **µL of 1M KOH_{aq}** | **mL of H₂O** | **pH 12h** | **pH 24h** | **pH 48h** | **pH 1w** | **Solubility** |
|---|---|---|---|---|---|---|---|---|---|
| 100 | 40 °C | 0.84 / 1 | 439 | 2.55 | 7.5 | 7.2 | 7.5 | 7.5 | a |
| 100 | 40 °C | 0.86 / 1 | 450 | 2.54 | 7.9 | 7.9 | 8.0 | 7.9 | c |
| 100 | 40 °C | 0.88 / 1 | 452 | 2.49 | 8.0 | 8.0 | 8.1 | 8.0 | c |
| 100 | 60 °C | 0.84 / 1 | 432 | 2.51 | 7.8 | 7.8 | 8.1 | 7.8 | c |
| 100 | 60 °C | 0.86 / 1 | 446 | 2.52 | 7.9 | 7.9 | 8.1 | 7.9 | c |
| 100 | 60 °C | 0.88 / 1 | 457 | 2.52 | 8.1 | 8.0 | 8.2 | 8.1 | c |

| | | | | | | | | | |
|---|---|---|---|---|---|---|---|---|---|
| **a:** particles in suspension; c: clear solution. | | | | | | | | | |

### Results

Samples of 20 mg/mL of Letermovir Free Base with KOH present always particles in suspension except for the sample with 1 equivalent of KOH (only some particles remain above the solution, in the air-water interphase).

All the samples of 100 mg/mL of Letermovir Free Base with KOH are completely dissolved (only some particles remain above the solution, in the air-water interphase the first 2 days).

By increasing the temperature, the effects are slightly different:
▪ 40 °C
   - Samples of 20 mg/mL of Letermovir Free Base with 0.84, 0.86 and 0.88 equivalents of KOH present particles in suspension and the appearance is cloudy.
   - Samples of 100 mg/mL of Letermovir Free Base with 0.84, 0.86 and 0.88 equivalents of KOH present particles in suspension.
▪ 60 °C
   - Sample of 20 mg/mL of Letermovir Free Base with 0.84 equivalents of KOH presents particles in suspension.
   - Samples of 20 mg/mL of Letermovir Free Base with 0.86 and 0.88 equivalents of KOH and samples of 100 mg/mL of Letermovir Free Base with 0.84, 0.86 and 0.88 equivalents of KOH are clear solutions from the first day and after 1 week.

### Example 2. Lyophilization after 7 days and reconstitution in water

### a) Initial drying

14 samples of letermovir free base were prepared by weighing the substance and drying in a vacuum oven at 90 °C (ca. 5 mbar) overnight in order to remove residual water and to avoid weighing errors to calculate the equivalents of KOH (Table 8).

Samples of ca. 80 mg and 300 mg were weighed to be dissolved in 4 mL and 3 mL, respectively, to prepare 20 mg/mL and 100 mg/mL solutions.

**Table 8. Initial drying process of 14 samples of letermovir free base.**

| **Planned Letermovir Free Base Concentration [mg/ml]** | **Planned KOH/Letermovir Molar Ratio** | **Starting material, g** | **Sample lost, g** | **Final weight, g** |
|---|---|---|---|---|
| 20 | 0.80 / 1 | 0.0806 | 0.0021 | 0.0785 |
| 20 | 0.82 / 1 | 0.0805 | 0.0022 | 0.0783 |
| 20 | 0.84 / 1 | 0.0808 | 0.0026 | 0.0782 |
| 20 | 0.86 / 1 | 0.0807 | 0.0023 | 0.0784 |
| 20 | 0.88 / 1 | 0.0803 | 0.0020 | 0.0783 |
| 20 | 0.90 / 1 | 0.0816 | 0.0021 | 0.0795 |
| 20 | 1 / 1 | 0.0803 | 0.0020 | 0.0783 |
| 100 | 0.80 / 1 | 0.3016 | 0.0057 | 0.2959 |
| 100 | 0.82 / 1 | 0.3008 | 0.0059 | 0.2949 |
| 100 | 0.84 / 1 | 0.3018 | 0.0061 | 0.2957 |
| 100 | 0.86 / 1 | 0.3013 | 0.0060 | 0.2953 |
| 100 | 0.88 / 1 | 0.3007 | 0.0059 | 0.2948 |
| 100 | 0.90 / 1 | 0.3019 | 0.0060 | 0.2959 |
| 100 | 1 / 1 | 0.3011 | 0.0059 | 0.2952 |

### b) Preparation of suspensions/solutions and analysis of solubility

Procedure: The corresponding amount of water and the respective equivalents of 1 M aqueous KOH were added to each sample. The suspensions were stirred at room temperature and the solubility and precipitation effects were checked (Table 9).

**Table 9. Preparation of samples of letermovir free base in water and with different equivalents of KOH.**

| **Letermovir Free Base Concentration [mg/ml]** | **KOH/Letermovir Molar Ratio** | **µL of 1M KOH_{aq}** | **mL of H₂O** |
|---|---|---|---|
| 20 | 0.80 / 1 | 110 | 3.82 |
| 20 | 0.82 / 1 | 112 | 3.80 |
| 20 | 0.84 / 1 | 115 | 3.80 |
| 20 | 0.86 / 1 | 118 | 3.80 |
| 20 | 0.88 / 1 | 120 | 3.79 |
| 20 | 0.90 / 1 | 125 | 3.85 |
| 20 | 1 / 1 | 137 | 3.78 |
| 100 | 0.80 / 1 | 413 | 2.55 |
| 100 | 0.82 / 1 | 422 | 2.53 |
| 100 | 0.84 / 1 | 434 | 2.52 |
| 100 | 0.86 / 1 | 444 | 2.51 |
| 100 | 0.88 / 1 | 453 | 2.49 |
| 100 | 0.90 / 1 | 465 | 2.49 |
| 100 | 1 / 1 | 516 | 2.44 |

### Solubility over 7 days

After 1 week, the precipitate remains in samples of 20 mg/mL with 0.8, 0.82, 0.84 and 0.86 equivalents of KOH. In sample of 20 mg/mL with 0.88 equivalents of KOH a small amount of solid is observed and samples of 20 mg/mL with 0.9 and 1 equivalent of KOH are clear solutions. All the samples of 100 mg/mL are clear solutions after a week.

### c) Lyophilisation and reconstitution in water

After 1 week the samples were lyophilised.

### Procedure:

Samples of 20 mg/mL: an aliquot of 3 mL was placed in the freezer for 2 hours. The samples were frozen using liquid nitrogen and the freeze drying process was performed over 2 days (average vacuum ca. 0.05 mbar, temperature ca. -86 °C). A white amorphous powder was obtained. The obtained solid was analysed by PXRD which confirmed the amorphous nature of the freeze-dried material. The obtained solid was solubilized with ca. 3 mL of water in order to get a final concentration of 20 mg/mL and checked for precipitation and pH (Table 8).

Samples of 100 mg/mL: an aliquot of 2.6 mL was placed in the freezer for 2 hours. The samples were frozen using liquid nitrogen and the freeze drying process was performed over 2 days (average vacuum ca. 0.05 mbar, temperature ca. -86 °C). A white amorphous powder was obtained. The obtained solid was analysed by PXRD which confirmed the amorphous nature of the freeze-dried material. The obtained solid was solubilized with ca. 13 mL of water in order to get a final concentration of 100 mg/mL and checked for precipitation and pH (Table 10).

**Table 10. Lyophilization and reconstitution in water**

| **Initial Letermovir Free Base Concentration [mg/ml]** | **Initial KOH/Letermovir Molar Ratio** | **Final concentration [mg/ml]** | **pH 24h** | **Solubility** |
|---|---|---|---|---|
| 20 | 0.80 / 1 | 20 | 7.5 | Not soluble |
| 20 | 0.82 / 1 | 20 | 7.5 | Not soluble |
| 20 | 0.84 / 1 | 20 | 7.5 | Not soluble |
| 20 | 0.86 / 1 | 20 | 7.5 | Not soluble |
| 20 | 0.88 / 1 | 20 | 7.4 | Almost soluble |
| 20 | 0.90 / 1 | 20 | 7.5 | Clear solution |
| 20 | 1 / 1 | 20 | 7.7 | Clear solution |
| 100 | 0.80 / 1 | 20 | 7.5 | Not soluble |
| 100 | 0.82 / 1 | 20 | 7.5 | Not soluble |
| 100 | 0.84 / 1 | 20 | 7.5 | Not soluble |
| 100 | 0.86 / 1 | 20 | 7.5 | Not soluble |
| 100 | 0.88 / 1 | 20 | 7.5 | Clear solution |
| 100 | 0.90 / 1 | 20 | 7.6 | Clear solution |
| 100 | 1 / 1 | 20 | 7.8 | Clear solution |

The behaviour of the samples with an initial and final concentration of 20 mg/mL is the same before and after the lyophilisation and reconstitution in water. However, the solubility of the samples with an initial concentration of 100 mg/mL is lower when reconstituted in water at a concentration of 20 mg/mL. Only the samples with a larger amount of KOH dissolve completely.

### Example 3. Lyophilization after 7 days and reconstitution in Ringer's Lactate solution

### a) Initial drying

14 samples of letermovir free base were prepared by weighing the substance and drying in a vacuum oven at 90 °C (ca. 5 mbar) overnight in order to remove residual water and to avoid weighing errors to calculate the equivalents of KOH (Table 11).

Samples of ca. 80 mg and 300 mg were weighed to be dissolved in 4 mL and 3 mL, respectively, to prepare 20 mg/mL and 100 mg/mL solutions.

**Table 11. Initial drying process of 14 samples of letermovir Free Base.**

| **Planned Letermovir Free Base Concentration [mg/ml]** | **Planned KOH/Letermovir Molar Ratio** | **Starting material, g** | **Sample lost, g** | **Final weight, g** |
|---|---|---|---|---|
| 20 | 0.80 / 1 | 0.0812 | 0.0021 | 0.0791 |
| 20 | 0.82 / 1 | 0.0809 | 0.0021 | 0.0788 |
| 20 | 0.84 / 1 | 0.0816 | 0.0019 | 0.0797 |
| 20 | 0.86 / 1 | 0.0809 | 0.0020 | 0.0789 |
| 20 | 0.88 / 1 | 0.0805 | 0.0020 | 0.0785 |
| 20 | 0.90 / 1 | 0.0809 | 0.0016 | 0.0793 |
| 20 | 1 / 1 | 0.0808 | 0.0016 | 0.0792 |
| 100 | 0.80 / 1 | 0.3004 | 0.0058 | 0.2946 |
| 100 | 0.82 / 1 | 0.3004 | 0.0056 | 0.2948 |
| 100 | 0.84 / 1 | 0.3019 | 0.0057 | 0.2962 |
| 100 | 0.86 / 1 | 0.3020 | 0.0058 | 0.2962 |
| 100 | 0.88 / 1 | 0.3013 | 0.0058 | 0.2955 |
| 100 | 0.90 / 1 | 0.3022 | 0.0058 | 0.2964 |
| 100 | 1 / 1 | 0.3010 | 0.0053 | 0.2957 |

### b) Preparation of suspensions/solutions and analysis of solubility

Procedure: The corresponding amount of water and the respective equivalents of 1 M aqueous KOH were added to each sample. The suspensions were stirred at room temperature and the solubility and precipitation effects were checked (Table 12).

**Table 12. Preparation of samples of letermovir free base in water and with different equivalents of KOH.**

| **Letermovir Free Base Concentration [mg/ml]** | **KOH/Letermovir Molar Ratio** | **µL of 1M KOH_{aq}** | **mL of H₂O** |
|---|---|---|---|
| 20 | 0.80 / 1 | 111 | 3.84 |
| 20 | 0.82 / 1 | 113 | 3.83 |
| 20 | 0.84 / 1 | 117 | 3.87 |
| 20 | 0.86 / 1 | 119 | 3.83 |
| 20 | 0.88 / 1 | 121 | 3.80 |
| 20 | 0.90 / 1 | 125 | 3.84 |
| 20 | 1 / 1 | 138 | 3.82 |
| 100 | 0.80 / 1 | 412 | 2.53 |
| 100 | 0.82 / 1 | 422 | 2.53 |
| 100 | 0.84 / 1 | 435 | 2.53 |
| 100 | 0.86 / 1 | 445 | 2.52 |
| 100 | 0.88 / 1 | 454 | 2.50 |
| 100 | 0.90 / 1 | 466 | 2.50 |
| 100 | 1 / 1 | 517 | 2.44 |

### Solubility over 7 days

After 1 week, the precipitate remains in samples of 20 mg/mL with 0.8, 0.82, 0.84 and 0.86 equivalents of KOH. In sample of 20 mg/mL with 0.88 equivalents of KOH a small amount of solid is observed and samples of 20 mg/mL with 0.9 and 1 equivalent of KOH are clear solutions. All the samples of 100 mg/mL are clear solutions after a week.

### c) Lyophilisation and reconstitution in Ringer's Lactate

After 1 week the samples were lyophilised.

### Procedure:

Samples of **20 mg/mL:** an aliquot of 3 mL was placed in the freezer for 2 hours. The samples were frozen using liquid nitrogen and the freeze drying process was performed over 2 days (average vacuum ca. 0.05 mbar, temperature ca. -86 °C). A white amorphous powder was obtained. The obtained solid was analysed by PXRD which confirmed the amorphous nature of the freeze-dried material. The obtained solid was solubilized with ca. 3 mL of Ringer's Lactate solution in order to get a final concentration of 20 mg/mL and checked for precipitation and pH (Table 13).

Samples of **100 mg/mL:** an aliquot of 2.6 mL was placed in the freezer for 2 hours. The samples were frozen using liquid nitrogen and the freeze drying process was performed over 2 days (average vacuum ca. 0.05 mbar, temperature ca. -86 °C). A white amorphous powder was obtained. The obtained solid was analysed by PXRD which confirmed the amorphous nature of the freeze-dried material. The obtained solid was solubilized with ca. 13 mL of Ringer's Lactate solution in order to get a final concentration of 100 mg/mL and checked for precipitation and pH (Table 13).

**Table 13. Lyophilization and reconstitution in Ringer's Lactate.**

| **Initial Letermovir Free Base Concentration [mg/ml]** | **Initial KOH/Letermovir Molar Ratio** | **Final concentration [mg/ml]** | **pH** | **Solubility** |
|---|---|---|---|---|
| 20 | 0.80 / 1 | 20 | 7.3 | Clear solution |
| 20 | 0.82 / 1 | 20 | 7.3 | Clear solution |
| 20 | 0.84 / 1 | 20 | 7.3 | Clear solution |
| 20 | 0.86 / 1 | 20 | 7.3 | Clear solution |
| 20 | 0.88 / 1 | 20 | 7.3 | Clear solution |
| 20 | 0.90 / 1 | 20 | 7.4 | Clear solution |
| 20 | 1 / 1 | 20 | 7.6 | Clear solution |
| 100 | 0.80 / 1 | 20 | 7.3 | Clear solution |
| 100 | 0.82 / 1 | 20 | 7.3 | Clear solution |
| 100 | 0.84 / 1 | 20 | 7.4 | Clear solution |
| 100 | 0.86 / 1 | 20 | 7.4 | Clear solution |
| 100 | 0.88 / 1 | 20 | 7.5 | Clear solution |
| 100 | 0.90 / 1 | 20 | 7.4 | Clear solution |
| 100 | 1 / 1 | 20 | 7.7 | Clear solution |

In the samples with a concentration of 20 mg/mL the behaviour before and after the lyophilisation and reconstitution in Ringer's lactate is slightly different. While the samples with a lower amount of KOH did not dissolve completely before the lyophilisation, all dissolve in the reconstitution, giving clear solutions.

**Example 4. Lyophilization after 7 days and reconstitution in Glucose aqueous 5 % solution**

### a) Initial drying

14 samples of letermovir free base were prepared by weighing the substance and drying in a vacuum oven at 90 °C (ca. 5 mbar) overnight in order to remove residual water and to avoid weighing errors to calculate the equivalents of KOH (Table 14).

Samples of ca. 80 mg and 300 mg were weighed to be dissolved in 4 mL and 3 mL, respectively, to prepare 20 mg/mL and 100 mg/mL solutions.

**Table 14. Initial drying process of 14 samples of letermovir free base.**

| **Planned Letermovir Free Base Concentration [mg/ml]** | **Planned KOH/Letermovir Molar Ratio** | **Starting material, g** | **Sample lost, g** | **Final weight, g** |
|---|---|---|---|---|
| 20 | 0.80 / 1 | 0.0814 | 0.0017 | 0.0797 |
| 20 | 0.82 / 1 | 0.0802 | 0.0018 | 0.0784 |
| 20 | 0.84 / 1 | 0.0808 | 0.0021 | 0.0787 |
| 20 | 0.86 / 1 | 0.0809 | 0.0019 | 0.0790 |
| 20 | 0.88 / 1 | 0.0811 | 0.0019 | 0.0792 |
| 20 | 0.90 / 1 | 0.0816 | 0.0021 | 0.0795 |
| 20 | 1 / 1 | 0.0806 | 0.0021 | 0.0785 |
| 100 | 0.80 / 1 | 0.3004 | 0.0053 | 0.2951 |
| 100 | 0.82 / 1 | 0.3022 | 0.0055 | 0.2967 |
| 100 | 0.84 / 1 | 0.3013 | 0.0057 | 0.2956 |
| 100 | 0.86 / 1 | 0.3010 | 0.0055 | 0.2955 |
| 100 | 0.88 / 1 | 0.3024 | 0.0054 | 0.2970 |
| 100 | 0.90 / 1 | 0.3020 | 0.0055 | 0.2965 |
| 100 | 1 / 1 | 0.3026 | 0.0055 | 0.2971 |

### b) Preparation of suspensions/solutions and analysis of solubility

Procedure: the corresponding amount of water and the respective equivalents of 1 M aqueous KOH were added to each sample. The suspensions were stirred at room temperature and the solubility and precipitation effects were checked (Table 15).

**Table 15. Preparation of samples of letermovir free base in water and with different equivalents of KOH.**

| **Letermovir Free Base Concentration [mg/ml]** | **KOH/Letermovir Molar Ratio** | **µL of 1M KOH_{aq}** | **mL of H₂O** |
|---|---|---|---|
| 20 | 0.80 / 1 | 111 | 3.87 |
| 20 | 0.82 / 1 | 112 | 3.81 |
| 20 | 0.84 / 1 | 116 | 3.82 |
| 20 | 0.86 / 1 | 119 | 3.83 |
| 20 | 0.88 / 1 | 122 | 3.84 |
| 20 | 0.90 / 1 | 125 | 3.85 |
| 20 | 1 / 1 | 137 | 3.79 |
| 100 | 0.80 / 1 | 412 | 2.54 |
| 100 | 0.82 / 1 | 425 | 2.54 |
| 100 | 0.84 / 1 | 434 | 2.52 |
| 100 | 0.86 / 1 | 444 | 2.51 |
| 100 | 0.88 / 1 | 457 | 2.51 |
| 100 | 0.90 / 1 | 466 | 2.50 |
| 100 | 1 / 1 | 519 | 2.45 |

### Solubility over 7 days

After 1 week, the precipitate remains in samples of 20 mg/mL with 0.8, 0.82, 0.84 and 0.86 equivalents of KOH. In sample of 20 mg/mL with 0.88 equivalents of KOH a small amount of solid is observed and samples of 20 mg/mL with 0.9 and 1 equivalent of KOH are clear solutions. All the samples of 100 mg/mL are clear solutions after a week.

### c) Lyophilisation and reconstitution in Glucose 5% aqueous solution

After 1 week the samples were lyophilised.

### Procedure:

Samples of **20 mg/mL:** an aliquot of 3 mL was placed in the freezer for 2 hours. The samples were frozen using liquid nitrogen and the freeze drying process was performed over 2 days (average vacuum ca. 0.05 mbar, temperature ca. -86 °C). A white amorphous powder was obtained. The obtained solid was analysed by PXRD which confirmed the amorphous nature of the freeze-dried material. The obtained solid was solubilized with ca. 3 mL of a 5% glucose solution in order to get a final concentration of 20 mg/mL and checked for precipitation and pH (Table 16).

Samples of **100 mg/mL:** an aliquot of 2.6 mL was placed in the freezer for 2 hours. The samples were frozen using liquid nitrogen and the freeze drying process was performed over 2 days (average vacuum ca. 0.05 mbar, temperature ca. -86 °C). A white amorphous powder was obtained. The obtained solid was analysed by PXRD which confirmed the amorphous nature of the freeze-dried material. The obtained solid was solubilized with ca. 13 mL of a 5% glucose solution in order to get a final concentration of 100 mg/mL and checked for precipitation and pH (Table 16).

**Table 16. Lyophilization and reconstitution in glucose 5% aqueous solution.**

| **Letermovir Free Base Initial Concentration [mg/ml]** | **Initial KOH/Letermovir Molar Ratio** | **Final concentration [mg/ml]** | **pH** | **Solubility** |
|---|---|---|---|---|
| 20 | 0.80 / 1 | 20 mg/mL | 7.4 | Not soluble |
| 20 | 0.82 / 1 | 20 mg/mL | 7.5 | Not soluble |
| 20 | 0.84 / 1 | 20 mg/mL | 7.5 | Almost soluble |
| 20 | 0.86 / 1 | 20 mg/mL | 7.5 | Almost soluble |
| 20 | 0.88 / 1 | 20 mg/mL | 7.4 | Clear solution |
| 20 | 0.90 / 1 | 20 mg/mL | 7.5 | Clear solution |
| 20 | 1 / 1 | 20 mg/mL | 7.7 | Clear solution |
| 100 | 0.80 / 1 | 20 mg/mL | 7.5 | Not soluble |
| 100 | 0.82 / 1 | 20 mg/mL | 7.5 | Not soluble |
| 100 | 0.84 / 1 | 20 mg/mL | 7.5 | Not soluble |
| 100 | 0.86 / 1 | 20 mg/mL | 7.4 | Clear solution |
| 100 | 0.88 / 1 | 20 mg/mL | 7.4 | Clear solution |
| 100 | 0.90 / 1 | 20 mg/mL | 7.6 | Clear solution |
| 100 | 1 / 1 | 20 mg/mL | 7.8 | Clear solution |

The reconstitution in a 5% Glucose solution has a similar result as the reconstitution in water. Only the samples that contain a larger amount of KOH dissolve completely.

## Claims

1. A pharmaceutical composition comprising letermovir of formula (I), and potassium ions 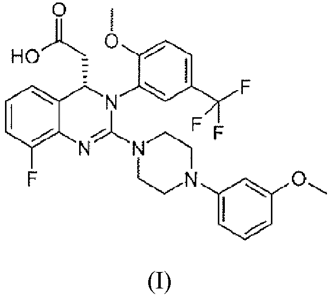 wherein the pharmaceutical composition
• comprises the potassium ions in a molar ratio to letermovir in the range of from 0.80 to < 1.00 : 1.00, preferably of from 0.88 to < 1.00 : 1.00, more preferably of from 0.90 to < 1.00 : 1.00; and
• is capable of exhibiting a pH in the range of from 7 to 8, preferably 7.4 to 7.8, when said pharmaceutical composition is dissolved in water in a concentration range of from 20 to 100 mg/mL with respect to letermovir; and
• is essentially free from complexing solubilizing agents selected from the group consisting of PEG, lysine, arginine, a cyclodextrin, in particular a hydroxypropyl-beta-cyclodextrin (HPBCD),
wherein the potassium ions are contained in the form of a solution of potassium hydroxide (KOH), preferably an aqueous solution of KOH.

2. The pharmaceutical composition according to claim 1, further comprising at least one excipient selected from the group consisting of a carbohydrate, in particular selected from sucrose and mannitol, an amino acid, in particular phenylalanine, a polyalkoxy compound, in particular a poloxamer, more particular poloxamer 188, and a polyvinylpyrrolidone (PVP), in particular PVP PF12.

3. The pharmaceutical composition according to claim 1, wherein the pharmaceutical composition is essentially free from complexing solubilizing agents.

4. The pharmaceutical composition according to claim 1 or 2, wherein the pharmaceutical composition comprises a polyalkoxy compound, in particular a poloxamer, more particular poloxamer 188, and is essentially free from other complexing solubilizing agents.

5. The pharmaceutical composition according to claim 2, wherein the excipient is mannitol or sucrose or a combination thereof.

6. The pharmaceutical composition according to any one of claims 1 to 5, further comprising a buffer, preferably Tris hydroxy aminomethane (Tris).

7. A method of producing the pharmaceutical composition as defined in any one of claims 1 to 6, comprising the following steps:
i) providing a solution of letermovir and potassium ions, wherein the molar ratio of potassium ions to letermovir is in the range of from 0.80 to < 1.00 : 1.00, preferably of from 0.88 to < 1.00 : 1.00, more preferably of from 0.90 to < 1.00 : 1.00, and optionally at least one excipient selected from the group consisting of a carbohydrate, in particular sucrose or mannitol, an amino acid, in particular phenylalanine, a polyalkoxy compound, in particular a poloxamer, more particular poloxamer 188, and a polyvinylpyrrolidone (PVP), in particular PVP PF12, wherein the potassium ions are provided in the form of a KOH solution, preferably an aqueous KOH solution
ii) if needed adjusting the pH of the solution obtained in step i) to a range of from 7 to 8, preferably from 7.4 to 7.8, preferably with HCl
iii) optionally filtering said solution.

8. The method according to claim 7, further comprising the subsequent additional step of freeze-drying the obtained solution to provide a lyophilizate.

9. The method according to claim 8, further comprising the subsequent additional step of reconstituting the lyophilizate in a first parenterally acceptable diluent to provide a reconstituted solution in a concentration range of from 20 to 100 mg/mL with respect to letermovir and optionally subsequently diluting said reconstituted solution with a second parenterally acceptable diluent to a final concentration which is acceptable for injection or infusion, wherein said first and said second parenterally acceptable diluents can be the same or different.

10. The pharmaceutical composition according to any one of claims 1 to 6 for use in a method of treatment and/or prevention of diseases, in particular of virus infections, preferably human cytomegalovirus (HCMV) infections or infections with another member of the herpes viridae group.

## Patentansprüche

1. Pharmazeutische Zusammensetzung umfassend Letermovir der Formel (1), und Kaliumionen 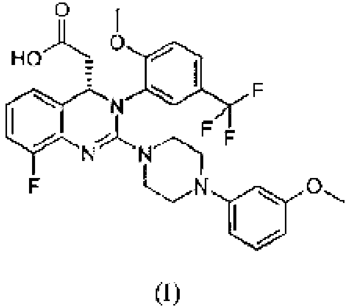 wobei die pharmazeutische Zusammensetzung
• Kaliumionen in einem molaren Verhältnis zu Letermovir im Bereich von 0,80 bis < 1,00 : 1,00, bevorzugt von 0,88 bis < 1,00 : 1,00, weiter bevorzugt von 0,90 bis < 1,00 : 1,00 umfasst; und
• fähig ist, einen pH-Wert im Bereich von 7 bis 8, bevorzugt 7,4 bis 7,8 aufzuweisen, wenn die pharmazeutische Zusammensetzung in einem Konzentrationsbereich von 20 bis 100 mg/ml in Bezug auf Letermovir in Wasser gelöst wird; und
• im Wesentlichen frei ist von komplexbildenden solubilisierenden Wirkstoffen, ausgewählt aus der Gruppe bestehend aus PEG, Lysin, Arginin, einem Cyclodextrin, insbesondere einem Hydroxypropyl-beta-cyclodextrin (HPBCD),
wobei die Kaliumionen in Form einer Lösung von Kaliumhydroxid (KOH) enthalten sind, bevorzugt eine wässrige Lösung von KOH.

2. Pharmazeutische Zusammensetzung nach Anspruch 1, weiterhin umfassend mindestens einen Hilfsstoff, ausgewählt aus der Gruppe bestehend aus einem Kohlenhydrat, insbesondere ausgewählt aus Saccharose und Mannitol, einer Aminosäure, insbesondere Phenylalanin, einer Polyalkoxy-Verbindung, insbesondere einem Poloxamer, weiter insbesondere Poloxamer 188, und einem Polyvinylpyrrolidon (PVP), insbesondere PVP PF 12.

3. Pharmazeutische Zusammensetzung nach Anspruch 1, wobei die pharmazeutische Zusammensetzung im Wesentlichen frei von komplexbildenden solubilisierenden Wirkstoffen ist.

4. Pharmazeutische Zusammensetzung nach Anspruch 1 oder 2, wobei die pharmazeutische Zusammensetzung eine Polyalkoxyverbindung umfasst, insbesondere ein Poloxamer, weiter insbesondere ein Poloxamer 188, und im Wesentlichen frei von anderen komplexbildenden solubilisierenden Wirkstoffen ist.

5. Pharmazeutische Zusammensetzung nach Anspruch 2, wobei der Hilfsstoff Mannitol oder Saccharose oder eine Kombination davon ist.

6. Pharmazeutische Zusammensetzung nach einem der Ansprüche 1 bis 5, weiterhin umfassend einen Puffer, bevorzugt Trishydroxyaminomethan (Tris).

7. Verfahren zur Herstellung der pharmazeutischen Zusammensetzung wie in einem der Ansprüche 1 bis 6 definiert, wobei das Verfahren die folgenden Schritte umfasst:
i) Bereitstellen einer Lösung aus Letermovir und Kaliumionen, wobei das molare Verhältnis der Kaliumionen zu Letermovir im Bereich von 0,80 bis < 1,00 : 1,00, bevorzugt von 0,88 bis < 1,00 : 1,00, weiter bevorzugt von 0,90 bis < 1,00 : 1,00 liegt, und wahlweise mindestens eines Hilfsstoffs, ausgewählt aus der Gruppe bestehend aus einem Kohlenhydrat, insbesondere Saccharose oder Mannitol, einer Aminosäure, insbesondere Phenylalanin, einer Polyalkoxy-Verbindung, insbesondere eines Poloxamers, weiter insbesondere Poloxamer 188, und eines Polyvinylpyrrolidons (PVP), insbesondere PVP PF 12, wobei die Kaliumionen in Form einer KOH-Lösung, bevorzugt einer wässrigen KOH-Lösung, bereitgestellt werden
ii) falls nötig, Anpassen des pH-Wertes der in Schritt i) erhaltenen Lösung an einen Bereich von 7 bis 8, bevorzugt von 7,4 bis 7,8, bevorzugt mit HCl
iii) wahlweise Filtern der Lösung.

8. Verfahren nach Anspruch 7, weiterhin umfassend den nachfolgenden zusätzlichen Schritt des Gefriertrocknens der erhaltenen Lösung, um ein Lyophilisat bereitzustellen.

9. Verfahren nach Anspruch 8, weiterhin umfassend den nachfolgenden zusätzlichen Schritt der Rekonstitution des Lyophilisats in einem ersten parenteral verträglichen Verdünnungsmittel, um eine rekonstituierte Lösung in einem Konzentrationsbereich von 20 bis 100 mg/ml in Bezug auf Letermovir bereitzustellen und wahlweise anschließend Verdünnen der rekonstituierten Lösung mit einem zweiten parenteral verträglichen Verdünnungsmittel auf eine Endkonzentration, die zur Injektion oder Infusion geeignet ist, wobei das erste und zweite parenteral verträgliche Verdünnungsmittel dasselbe oder verschieden sein kann.

10. Pharmazeutische Zusammensetzung nach einem der Ansprüche 1 bis 6 zur Verwendung in einem Verfahren zur Behandlung und/oder Prophylaxe von Krankheiten, insbesondere von Virusinfektionen, vorzugsweise von Infektionen mit dem humanen Cytomegalievirus (HCMV) oder einem anderen Vertreter der Gruppe der Herpesviridae.

## Revendications

1. Composition pharmaceutique comprenant du létermovir de formule (I) et des ions potassium 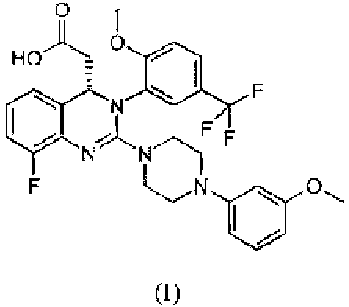 ladite composition pharmaceutique
• comprenant les ions potassium dont le rapport molaire au létermovir est compris entre 0,80 et < 1,00 : 1,00, de préférence entre 0,88 et < 1,00 : 1,00, et plus préférablement entre 0,90 et < 1,00 : 1,00; et
• pouvant présenter un pH compris entre 7 et 8, de préférence entre 7,4 et 7,8, lorsque ladite composition pharmaceutique est dissoute dans l'eau à une concentration comprise entre 20 et 100 mg/mL de létermovir; et
• étant essentiellement exempte d'agents solubilisants complexants choisis parmi le groupe constitué du PEG, de la lysine, de l'arginine, d'une cyclodextrine, en particulier d'une hydroxypropyl-bêta-cyclodextrine (HPBCD),
les ions potassium étant présents sous la forme d'une solution d'hydroxyde de potassium (KOH), de préférence sous la forme d'une solution aqueuse de KOH.

2. Composition pharmaceutique selon la revendication 1, comprenant en outre au moins un excipient choisi parmi le groupe constitué d'un glucide, en particulier choisi parmi le saccharose et le mannitol, d'un acide aminé, en particulier la phénylalanine, d'un composé polyalcoxy, en particulier un poloxamère, plus particulièrement le poloxamère 188, et d'une polyvinylpyrrolidone (PVP), en particulier la PVP PF 12.

3. Composition pharmaceutique selon la revendication 1, ladite composition pharmaceutique étant essentiellement exempte d'agents solubilisants complexants.

4. Composition pharmaceutique selon la revendication 1 ou 2, ladite composition pharmaceutique comprenant un composé polyalcoxy, en particulier un poloxamère, plus particulièrement le poloxamère 188, et étant essentiellement exempte d'autres agents solubilisants complexants.

5. Composition pharmaceutique selon la revendication 2, où l'excipient est le mannitol ou la saccharose, ou une combinaison de ceux-ci.

6. Composition pharmaceutique selon l'une des revendications 1 à 5, comprenant en outre un tampon, de préférence du Tris (hydroxyaminométhane) (Tris).

7. Procédé de préparation de la composition pharmaceutique telle que définie dans l'une des revendications 1 à 6, comprenant les étapes suivantes :
i) la mise à disposition d'une solution de létermovir et d'ions potassium, où le rapport molaire des ions potassium au létermovir est compris entre 0,80 et < 1,00 : 1,00, de préférence entre 0,88 et < 1,00 : 1,00, et plus préférablement entre 0,90 et < 1,00 : 1,00, et éventuellement d'au moins un excipient choisi parmi le groupe constitué d'un glucide, en particulier le saccharose ou le mannitol, d'un acide aminé, en particulier la phénylalanine, d'un composé polyalcoxy, en particulier un poloxamère, plus particulièrement le poloxamère 188, et d'une polyvinylpyrrolidone (PVP), en particulier la PVP PF12, les ions potassium étant apportés sous la forme d'une solution de KOH, de préférence une solution aqueuse de KOH,
ii) si nécessaire, l'ajustement du pH de la solution obtenue à l'étape i) dans une plage comprise entre 7 et 8, de préférence entre 7,4 et 7,8, de préférence à l'aide de HCl,
iii) éventuellement, la filtration de ladite solution.

8. Procédé selon la revendication 7, comprenant en outre l'étape supplémentaire consistant à lyophiliser la solution obtenue afin d'obtenir un lyophilisat.

9. Procédé selon la revendication 8, comprenant en outre l'étape supplémentaire consistant à reconstituer le lyophilisat dans un premier diluant acceptable par voie parentérale afin d'obtenir une solution reconstituée dans une plage de concentration comprise entre 20 et 100 mg/mL de létermovir et, éventuellement, à diluer ensuite ladite solution reconstituée avec un deuxième diluant acceptable par voie parentérale jusqu'à obtenir une concentration finale acceptable par injection ou une perfusion, lesdits premier et deuxième diluants acceptables par voie parentérale pouvant être identiques ou différents.

10. Composition pharmaceutique selon l'une des revendications 1 à 6, destinée à être utilisée dans un procédé de traitement et/ou de prévention de maladies, en particulier d'infections virales, de préférence d'infections par le cytomégalovirus humain (HCMV) ou d'infections par un autre membre du groupe des Herpesviridae.
